# EUROPEAN PATENT SPECIFICATION

(11) **EP 3 383 251 B1**
(45) Date of publication and mention of the grant of the patent: **08.01.2020**
(21) Application number: 16805062.3
(22) Date of filing: 29.11.2016
(51) Int. Cl.: A61B 5/00, A61B 5/107

(54) **FETAL POSITION MONITORING SYSTEM AND METHOD**
SYSTEM UND VERFAHREN ZUR ÜBERWACHUNG DER POSITION EINES FÖTUS
SYSTÈME ET PROCÉDÉ DE SURVEILLANCE DE POSITION FOETALE

(30) Priority: 30.11.2015 WO PCT/CN2015/095971; 07.01.2016 EP 16150399
(43) Date of publication of application: 10.10.2018
(73) Proprietor: Koninklijke Philips N.V., 5656 AE Eindhoven (NL)
(72) Inventor: TIAN, Cong, 5656 AE Eindhoven (NL); LI, Lin, 5656 AE Eindhoven (NL); WU, Yu, Qiang, 5656 AE Eindhoven (NL); WEN, Guang, Li, 5656 AE Eindhoven (NL); LOU, Xiaojun, 5656 AE Eindhoven (NL)
(74) Representative: van Oudheusden-Perset, Laure E.
(86) International application number: PCT/EP2016/079134
(87) International publication number: WO 2017/093251

(56) References cited:
- US-A1- 2014 249 436

## Description

### FIELD OF THE INVENTION

This invention relates to a fetal position monitoring system and method.

### BACKGROUND OF THE INVENTION

During pregnancy, mothers-to-be are often keen to know as much information about their baby as possible, for example including fetal position, fetal size and fetal movement, and they also wish to start to interact with their unborn baby as early as possible.

In obstetrics, Leopold's Maneuvers are a common and systematic way to determine the position of a fetus inside the woman's uterus after the 24th week of gestation.

There are four steps, known as the Fundal Grip, Lateral Grip, Pawlick's Grip, and Pelvic Grip.

Trained healthcare providers (HCPs) manually press certain abdominal areas with certain gestures and pressure, and thus determine the position of fetus and estimate the fetal size and weight based on the different morphology and mechanical properties of the fetal head, trunk, buttock, back and limbs. For example, under the 1st maneuver, fetal body parts can be differentiated by feeling that the head is hard, firm, round, and moves independently of the trunk, while the buttocks feel softer, and are symmetric. The shoulders and limbs have small bony projections and unlike the head, they move with the trunk.

More detailed examination about fetal development including fetal position and size may be conducted with ultrasonic imaging systems.

Both of these procedures are clinical solutions which are not applicable for home use because they require use of professional techniques, or require access to ultrasound diagnostic equipment.

People who are not well-trained in this (or other) manual procedure(s), such as the expectant mother or her family, could experience difficulties obtaining the desired results. Furthermore, these clinical solutions focus on diagnosis and are not applied to enable the expectant to interact with the fetus and improve the maternal-fetal bond.

A home-use assistive device to enable an untrained person to measure fetal movement is known from US2014/0249436.

There is therefore a need for a home-use assistive device to enable an un-trained person to measure fetal position and estimate fetal shape and size during their interaction with the fetus.

### SUMMARY OF THE INVENTION

The invention is defined by the claims.

According to examples in accordance with an aspect of the invention, there is provided a fetal position monitoring system, comprising:
a force and deformation sensor arrangement for obtaining resistance information of a plurality of local areas of the abdomen in response to an applied pressure the force and deformation sensor arrangement comprising a force and deformation sensor;
a data analysis processor for interpreting the resistance information in respect of the plurality of local areas, and for deriving a fetal position or fetal size from the interpretation; and
an output for providing output data to a an output device for presenting a representation of the fetal position or fetal size.

The resistance information is mechanical resistance information (rather than e.g. electrical resistance information) and it relates to the dynamic movement of the abdomen. This system provides a way of generating a representation of the fetal position and/or size which requires minimum medical knowledge of the user, and may thus be used by the expectant mother or her friends or relatives. The system may be used at home, as it does not rely on expensive imaging methodologies.

The system may enable the fetal size to be monitored.

It may additionally enable the fetal position to be determined. Once the fetal position is known, the mother can choose to interact using touch with specific body areas of the fetus. It also enables estimation of the fetal shape and/or fetal size.

The data analysis processor may further be for deriving fetal movement over time and the output is further for providing a representation of fetal movement. This provides additional information for the user of the system.

The system may be combined with other sensors, for example for monitoring the fetal heart beat or monitoring fetal movement.

The output device may be a display, and it may be a dedicated part of the system, or else it may be a remote device such as a tablet, laptop, personal computer, or mobile phone to which the data is transmitted from the output. The display may even comprise a projection system for displaying directly onto the abdomen of the expectant mother. The output device may instead or as well comprise an audio output, for example providing an audible indication of the size of the fetus.

In a first example, the force and deformation sensor arrangement comprises a glove, and the system comprises a controller for controlling the output for guiding the user of the system to apply pressure to the abdomen using the glove.

In this first example, the system may provide instructions to the user, for example relating to gestures to be followed, touching points and moving paths, durations of each hand action, intervals between actions and pressure levels which should be exerted. The instructions can be provided to the user by any suitable means such as an intelligent mobile device or directly projected upon the abdominal areas of the expectant mother. Duration and pressure of hand actions may be confined to be within pre-determined safety ranges. In this way, the user is involved in collecting the data, but the data interpretation is automated.

In a second example, the force and deformation sensor arrangement comprises an abdominal belt.

The use of a belt means that no skilled input is required by the user, and the system is almost fully automated.

The abdominal belt may comprise an array of inflatable portions for providing the applied pressure, with the force or deformation sensor arrangement associated with each inflatable portion.

The pressure is applied to different locations by the inflatable portions in a sequence which is controlled by, and therefore known to, the data analysis system.

In all examples, the force and deformation sensor comprises a sensor arrangement for measuring the force applied on belly and displacement of the belly induced by the applied force (i.e. by the user wearing the glove or by the belt). A stress can be calculated from force divided by the contacting area, and the compressive strain can be calculated from the displacement divided by thickness. Stress and strain may together provide more information about the nature of the tissue being pressed than a force or deformation measurement alone.

The force and deformation sensor may comprise a separate force sensor for measuring force and a separate deformation sensor for measuring deformation in response to an applied force.

The data analysis processor is for example adapted to derive a measure of hardness/compliance from the force and deformation measurements as the interpretation of the resistance information.

A hardness measure may be used to distinguish between different parts of the fetus.

The measure of hardness for example comprises a ratio of the deformation and force. This is related to a strain ratio. Thus, actual values of stress and strain do not need to be calculated to derive a measure (even if relative rather than absolute) of the desired strain ratio.

The data analysis processor is for example adapted to determine the location of abdominal areas adjacent or away from a fetal body part based on the interpretation of the resistance information. The fetal body part may be one or more of a head, a buttock and a back.

These different areas of the fetus have different hardness and position characteristics which can be recognized by the data analysis system. This list is not exhaustive, for example the limbs of the fetus may also be recognized. The data analysis processor is then adapted to determine the fetal position from the determined locations. The fetal shape and size may be determined as well as the general position and orientation.

Examples in accordance with another aspect of the invention provide a method of monitoring fetal position, comprising:
applying a force and deformation sensor arrangement to the abdominal area of an expectant mother;
obtaining resistance information of a plurality of local areas of the abdomen in response to an applied pressure;
interpreting the resistance information in respect of the plurality of local areas;
deriving a fetal position or size from the interpretation of the resistance information; and
providing a representation of the fetal position or fetal size.

This method generates an output of the fetal position or size without requiring significant medical knowledge of the user.

Obtaining resistance information may comprise measuring force and deformation. A measure of hardness may be obtained from a ratio of the deformation and force which is related to a ratio between stress and strain.

The location of abdominal areas adjacent or away from a fetal body part may be obtained based on the interpretation of the resistance information, wherein the fetal body part is one of a head, a buttock or a back. The fetal position may then be obtained from the head, buttock and/or back locations.

The force and deformation sensor arrangement in one example comprises a glove and the method comprises a user providing the applied pressure by hand through the glove.

In another example, the force and deformation sensor arrangement comprises an abdominal belt and the method comprises providing the applied pressure by inflating inflatable portions of the abdominal belt.

### BRIEF DESCRIPTION OF THE DRAWINGS

Examples of the invention will now be described in detail with reference to the accompanying drawings, in which:
Figure 1 shows the abdomen of a pregnant mother-to-be and shows the fetus;
Figure 2 shows a first implementation of a fetal position monitoring system;
Figure 3 shows a second implementation of a fetal position monitoring system;
Figure 4 shows the force (top graph) and deformation (bottom graph) when pressing the belly area, with no fetus beneath;
Figure 5 shows the force (top graph) and deformation (bottom graph) when pressing the belly area, with the fetus buttock beneath;
Figure 6 shows the force (top graph) and deformation (bottom graph) when pressing the belly area, with the fetus head beneath;
Figure 7 shows a first method of determining fetal shape, size or position; and
Figure 8 shows a second method of determining fetal shape, size or position.

### DETAILED DESCRIPTION OF THE EMBODIMENTS

The invention provides a fetal position monitoring system, comprising an abdominal belt or a glove comprising a force and deformation sensor arrangement for obtaining resistance information of a plurality of local areas of the abdomen in response to an applied pressure. A data analysis processor interprets the resistance information to derive a fetal position and/or fetal size and optionally also fetal movement and an output is provided giving a representation of the fetal position and/or fetal size and optionally also fetal movement.

This system provides a way of generating an output, such as a display of the fetal position or size which requires minimum medical knowledge of the user, and it may be used at home.

In a first implementation, the system instructs a user to measure the hardness of fetal body parts and the distribution of the fetal body parts by touching and pressing abdominal areas of the expectant with the aid of a biomechanical sensor based device. The instructions to the user for example identify one or more of:
touching points;
movement paths;
duration of different touch actions;
intervals between touch actions; and
pressure which should be exerted.

In a second implementation, an automated system is provided comprising a belt to be worn by the expectant mother.

The fetal position can be determined by mapping the distribution of the hardness in the touched abdominal area of the expectant mother. Different body parts of the fetus (e.g., head, back, limbs and buttocks) can be discriminated by the varying distribution and hardness.

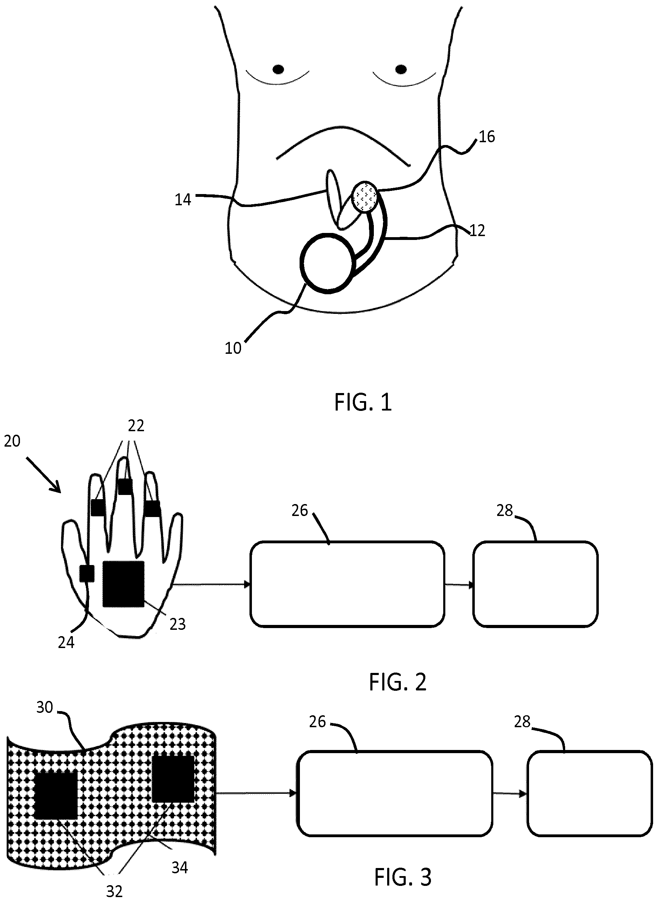

Figure 1 shows the abdomen of a pregnant mother-to-be and shows the fetus. Different parts of the fetus have different hardness and distribution of the hardness. For example, the head 10 is relatively hard and round, the back 12 is comparably hard but flat and broad (as shown by bold lines for the head and back), whereas the limbs 14 have small bony properties. The buttocks 16 are even less hard and symmetric.

In biomechanics, modulus or stress-strain curves can indicate the hardness of biomaterials. Thus, by calculation of the stress and strain of touched areas, for example using compression or deformation sensors, the hardness can be evaluated.

The fetal shape and size can be estimated by determining the area and length of different parts of the fetus. A reconstructed image of the fetal shape with information about the fetal position and size can then be displayed in real-time, or even projected directly upon the touched abdominal surface of the expectant mother. Images over time provide a representation of fetal movements.

The fetal response (e.g. fetal movement and fetal heart rate) can also be monitored and recorded. In this way, the mother-to-be may be able to learn what actions generate a response from the fetus. For example, the mother-to-be may use the system to learn what actions induce a desired response, such as evoking fetal movement. Such interactions may help to develop the bond between the fetus and the mother. The actions are not limited to application of pressure. For example, the system may be used to detect response of the fetus to other stimuli, such as sounds or movement of the mother-to-be.

Figure 2 shows the first implementation of the system.

The system comprises a force and deformation sensor arrangement for obtaining resistance information of a plurality of local areas of the abdomen in response to an applied pressure. In this example, the sensor arrangement comprises a glove 20 which has a plurality of sensing areas 22,23 provided on one or more fingers, and/or the palm and/or the thumb. The sensing areas serve multiple functions. One is to monitor the pressure applied by the user and the surface displacement in response to the pressure applied, and the other is to derive the resistance information of the body part being pressed. Different sensing areas may perform different functions, or the sensing areas may perform both functions.

The force sensor may be any compression sensor that can indicate the compression level e.g. piezoelectric sensors or Holzer pressure gauge sensors. The deformation sensor is any sensor that can indicate the surface displacement from the original position without compression e.g. flexi sensors, and fiber optical strain gauges.

A data analysis processor 26 is provided for interpreting the resistance information in respect of the plurality of local areas, and for deriving a fetal position from the interpretation. A display 28 is used to display a representation of the fetal position.

The user touches the abdominal area while wearing the glove 20. The sensing areas 22,23 for example each comprise one or both of a compression sensor for force measurement from which a stress can be determined, and a deformation sensor for deformation measurement from which strain may be determined. There may be one glove or a pair of gloves.

In one example, compression sensors 22 formed at the finger part of glove monitor the pressure exerted by the user's hand. When the pressure is out of a pre-determined safety range, a safety control function is triggered by the processor 26 to alert the user. The safety control function may comprise a vibrating motor 24 formed at the glove so that the user feels the safety warning. Alternatively, an audio or visual alarm may be provided. The compression sensors may comprise piezoelectric sensors.

Further compression sensors and deformation sensors 23 are provided as part of a fetal position determination area, in this example at the palm of the hand.

When wearing the glove or gloves, users can touch or press the abdominal area of the expectant by following instructions provided to the user on the display 28.

Both force and deformation of the touched area will then be measured and used for calculating a strain ratio which is an indicator of hardness and mapping the distribution of hardness.

The processor 26 may be part of the glove or it may be a separate device communicating with glove via wired or wireless methods. The processor 26 calculates the hardness of the touched area and thereby derives the information to be displayed. The display can be embodied in any device including a portable device such as a tablet or mobile phone, a computer, or a projection display for displaying directly upon the abdomen of the expectant mother.

By guiding the user through different actions to be taken when wearing the glove, the system enables untrained people to be able to determine the fetal position. However, there is still a need to follow instructions, so that the processor knows where the user has been touching the abdomen. The processor has to assume that the instructions have been correctly followed, so there is still the possibility of error if a user is not sufficiently skilled in following the instructions provided.

Figure 3 shows the second implementation, which provides a more fully automated system.

The system again comprises a force and deformation sensor arrangement for obtaining resistance information of a plurality of local areas of the abdomen in response to an applied pressure. In this example, the sensor arrangement comprises an abdominal belt 30 which has a plurality of sensing areas and inflatable air bags 32 to generate a pressure towards the abdomen of the expectant mother when wearing the belt. The system again comprises a processor 26 and display 28.

The force sensor may be of the same types as outlined above. Additionally, a gas-pressure meter associated with the inflatable portions may be used to derive the force applied.

One example of a suitable size of an individual airbag is a round airbag with a diameter between 5cm and 10cm, which thus has a similar area to the palm of the hand.

The space between the airbags should for example be less than 21cm, which is the Crown-rump length of the fetus at 24 weeks. A maximum pressure may for example be around 130KPa. The sensors are for example mounted over the airbag to have better contact with the belly area. If a gas pressure meter is used, it will of course be embodied in the airbags. The sensors may have a greater density in the fetus area. The data can be then used to interpret the boundary of each fetal part, e.g. head, back and buttock.

The pressure level and duration is controlled by the processor 26 within a predetermined safety range. A plurality of compression sensors (e.g. piezoelectric sensors, Holzer pressure gauge sensors and even gas pressure meters embodied in the inflatable portions) and deformation sensors (e.g. flexi sensors and fiber optical strain gauges), shown generally as the array of elements 34, is provided at the inner surface of the belt. In use, the airbags are inflated to exert pressure to the abdominal area covered by the belt, and at the same time both force and deformation of the touched areas are measured by the sensors 34.

The collected data is again used for calculating the strain ratio which is an indicator of hardness and for mapping distribution of hardness. The processor 26 is again either provided as part of the belt or it can be embodied in another separate device which communicates with the belt via wired or wireless methods. The processor calculates the hardness of the touched area and sends the relevant information to the display 28. The display can be embodied in any device including a portable device, computer, or a display directly on the belt itself, such as a flexible display.

In both implementations, the fetal shape and size can be estimated by calculating the area and length of different part of the fetus. The mother-to-be use the system to know the location and orientation of the fetus from the display, and uses her favorite way to interact with the fetus, such as tapping the head, petting the back, touching the hands or feet, playing music, or moving in a certain way, to meet the emotional needs of motherhood.

By using the system, the mother-to-be can also learn what interaction modes are preferred by the fetus, which give rise to positive reactions (e.g. fetal heart rate or fetal movement), and then use the interaction modes to please the fetus. The mother-to-be can repeat exactly the same interaction with the fetus every day, such as tapping the head, petting the back, touching the hands or feet, etc., and check the fetal response.

The system may, in a more elaborated implementation, further incorporate an ultrasound imaging system.

The functioning of the system has been tested, in particular by measuring the difference of force-deformation measurements as between the head, buttock and areas with no fetus beneath.

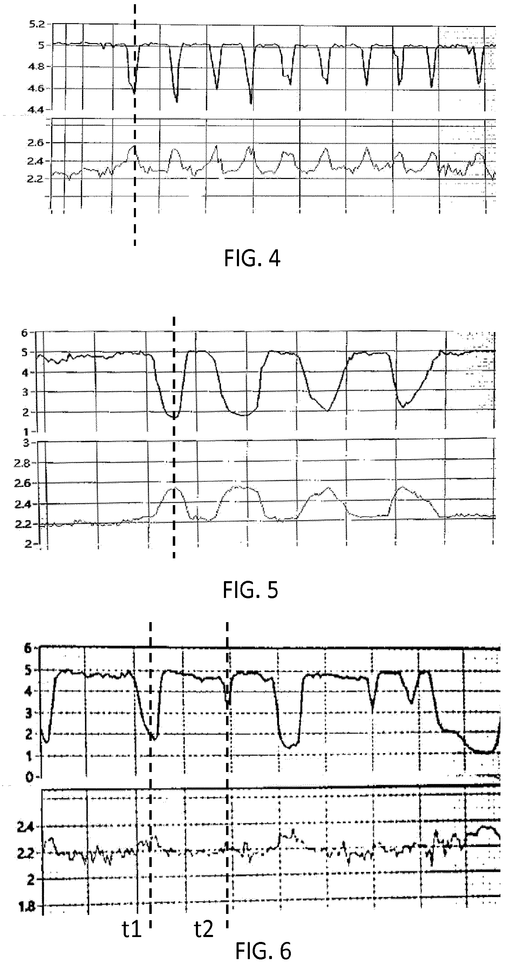

Figure 4 shows the force (top graph) and deformation (bottom graph) when pressing the belly area, with no fetus beneath, with a small applied force (1.87N) applied to an area of around 1 cm², so a stress of around 20kPa results. The x-axis indicates time.

A strain ratio may be defined as strain/stress x 100% which has a unit of Pa⁻¹.

The strain is equal to the measured deformation divided by thickness and stress is calculated from force divided by contacting area. By using the same piezoelectric sensors, the system is working under the same calibration conditions. The direct output value (in this case voltage) of both force and deformation measurements may thus be used to determine comparable values of the strain ratio.

Assuming a fixed contact area (around 1 cm²), and belly thickness, the strain ratio of strain/stress x 100% can be directly related to the deformation/force x 100% which may be provided as a direct output from the sensor arrangement. The strain ratio may thus be considered to be a dimensionless value for comparison purposes between different measurements.

The strain ratio is measured to be 31% for the belly area for the particular system configuration which has been used for testing.

Figure 5 shows the force (top graph) and deformation (bottom graph) when pressing the belly area, with the fetus buttock beneath, with a large applied stress (130KPa).

The strain ratio is 4.6% for the buttock area.

Figure 6 shows the force (top graph) and deformation (bottom graph) when pressing the belly area, with the fetus head beneath, with a large applied stress (130KPa) at time t1 and a medium applied stress (67KPa) at time t2.

The strain ratio is 1.6% for the head area with large stress and 1.3% with medium applied stress.

These simulations show that the fetal head, buttock and area in which no part of the fetus is present can be differentiated by the strain ratio. The fetal head is harder and consequently has a lower strain ratio as defined above (1-2%). The fetal buttock is softer and has therefore a relatively higher strain ratio (4-5%). The fetal position can be indicated by the identified fetal head and buttock.

The crown-rump length can be calculated by using position information of fetal head and fetal buttock. The fetal size can be estimated by using crown-rump length of the fetal shape.

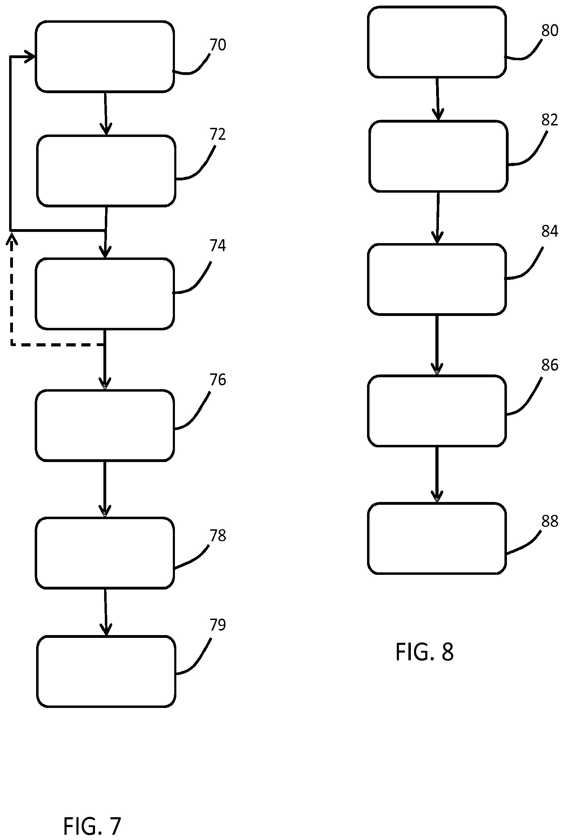

Figure 7 shows a method of determining fetal shape using the first (glove) implementation above.

In step 70 instructions are provided to the user as to how and where to press the abdomen while wearing the sensing glove. The instructions are provided using the display. The instructions to the user for example identify touching points at which contact is to be made, movement paths, duration of different touch actions, intervals between touch actions or the pressure level which should be exerted. These actions may together simulate Leopold's Maneuvers, but other sequences are also possible.

In step 72, the user applies the force and deformation sensor arrangement, in the form of the glove, to the abdominal area of an expectant mother.

This may be repeated with different positions, pressing forces, or movement shapes, so that steps 70 and 72 form a cycle of steps.

In step 74 resistance information is obtained for a plurality of local areas of the abdomen in response to the applied pressure. The resistance calculations may be performed at the end of sensing process (as shown) or they may carried out in real time, so that the calculations form part of the repeat loop. This is represented by the dotted loop arrow in Figure 7.

In step 76, the the resistance information is interpreted in respect of the plurality of local areas.

A fetal position and/or size is derived in step 78 from the interpretation of the resistance information, and a representation of the fetal position or fetal size and optionally also fetal movements is provided, e.g. displayed, in step 79.

Figure 8 shows a method of determining fetal shape using the second (abdominal belt) implementation above.

In step 80, the user applies the force and deformation sensor arrangement, in the form of the belt, to the abdominal area of an expectant mother.

In step 82 resistance information is obtained for a plurality of local areas of the abdomen in response to the applied pressure.

The applied pressures may be applied as a sequence or as a single large area measurement. This is then less time consuming and less effort and discomfort for the user. The system may however apply a pressure sequence. The belt may also be used for a belly massage.

In step 84, the resistance information is interpreted in respect of the plurality of local areas.

A fetal position or size is derived in step 86 from the interpretation of the resistance information, and a representation of the fetal position or fetal size and optionally also fetal movement is provided, e.g. displayed, in step 88.

## Claims

1. A fetal position monitoring system, comprising:
a force and deformation sensor arrangement (34) for obtaining resistance information of a plurality of local areas of the abdomen in response to an applied pressure, the force and deformation sensor arrangement (34) comprising a force and deformation sensor;
a data analysis processor (26) for interpreting the resistance information in respect of the plurality of local areas, and for deriving a fetal position or a fetal size from the interpretation; and
an output (28) for providing output data to a an output device for presenting a representation of the fetal position or fetal size.

2. A system as claimed in claim 1, wherein the data analysis processor is further for deriving fetal movement over time based on the fetal positions derived and the output device is further for providing a representation of the fetal movement.

3. A system as claimed in claim 1 or 2, wherein the force and deformation sensor arrangement comprises a glove associated with the force and deformation sensor, and wherein the system comprises a controller for controlling the output for guiding the user of the system to apply pressure to the abdomen using the glove.

4. A system as claimed in claim 1 or 2, wherein the force and deformation sensor arrangement comprises an abdominal belt.

5. A system as claimed in claim 4, wherein the abdominal belt comprises an array of inflatable portions for providing the applied pressure, each inflatable portion associated with the force and deformation sensor.

6. A system as claimed in claim 1, 3 or 5, wherein the force and deformation sensor comprises a force sensor for measuring force and a deformation sensor for measuring deformation in response to an applied force.

7. A system as claimed in claim 6, wherein the data analysis processor is adapted to derive a measure of hardness from a ratio of the deformation and force, as the interpretation of the resistance information.

8. A system as claimed in any preceding claim, wherein the data analysis processor is adapted to determine the location of abdominal areas adjacent or away from a fetal body part based on the interpretation of the resistance information, wherein the fetal body part is one of a head, a buttock or a back.

9. A system as claimed in claim 8, wherein the data analysis processor is adapted to determine the fetal position from the determined locations.

10. A method of monitoring fetal position, comprising:
applying a force and deformation sensor arrangement to the abdominal area of an expectant mother;
obtaining resistance information of a plurality of local areas of the abdomen in response to an applied pressure;
interpreting the resistance information in respect of the plurality of local areas;
deriving a fetal position or size from the interpretation of the resistance information; and
providing a representation of the fetal position or fetal size.

11. A method as claimed in claim 10, wherein obtaining resistance information comprises measuring force and deformation, and the method comprises deriving a measure of hardness from a ratio of the deformation and force,

12. A method as claimed in claim 10 or 11, comprising determining the location of abdominal areas adjacent or away from a fetal body part based on the interpretation of the resistance information, wherein the fetal body part is one or more of a head, a buttock and a back, and the method further comprises determining the fetal position from the head, buttock and/or back locations.

13. A method as claimed in any one of claims 10 to 12, wherein the force and deformation sensor arrangement comprises a glove and the method comprises a user providing the applied pressure through the glove.

14. A method as claimed in any one of claims 10 to 12, wherein the force and deformation sensor arrangement comprises an abdominal belt and the method comprises providing the applied pressure by inflating inflatable portions of the abdominal belt.

15. A method as claimed in any one of claims 10 to 14, further comprising deriving fetal movement over time, and providing a representation of the fetal movement.

## Patentansprüche

1. Fötuspositions-Überwachungssystem, umfassend:
eine Kraft- und Verformungssensoranordnung (34) zum Erhalten von Widerstandsinformationen einer Vielzahl von lokalen Bereichen des Abdomens als Reaktion auf einen angelegten Druck, wobei die Kraft- und Verformungssensoranordnung (34) einen Kraft- und Verformungssensor umfasst;
einen Datenanalyseprozessor (26) zum Interpretieren der Widerstandsinformation in Bezug auf die Vielzahl von lokalen Bereichen und zum Ableiten einer Fötusposition oder einer Fötusgröße aus der Interpretation; und
eine Ausgabe (28) zum Bereitstellen von Ausgabedaten an eine Ausgabevorrichtung zum Präsentieren einer Darstellung der Fötusposition oder Fötusgröße.

2. System nach Anspruch 1, wobei der Datenanalyseprozessor weiter zum Ableiten einer Fötusbewegung über die Zeit basierend auf den abgeleiteten Fötuspositionen dient und die Ausgabevorrichtung weiter zum Bereitstellen einer Darstellung der Fötusbewegung dient.

3. System nach Anspruch 1 oder 2, wobei die Kraft- und Verformungssensoranordnung einen Handschuh umfasst, der dem Kraft- und Verformungssensor zugeordnet ist, und wobei das System eine Steuerung zum Steuern der Ausgabe umfasst, um den Benutzer des Systems zum Anlegen von Druck an das Abdomen unter Verwendung des Handschuhs zu führen.

4. System nach Anspruch 1 oder 2, wobei die Kraft- und Verformungssensoranordnung einen Leibgurt umfasst.

5. System nach Anspruch 4, wobei der Leibgurt eine Anordnung von aufblasbaren Abschnitten zum Bereitstellen des angelegten Drucks umfasst, wobei jeder aufblasbare Abschnitt dem Kraft- und Verformungssensor zugeordnet ist.

6. System nach Anspruch 1, 3 oder 5, wobei der Kraft- und Verformungssensor einen Kraftsensor zum Messen der Kraft und einen Verformungssensor zum Messen der Verformung als Reaktion auf eine angelegte Kraft umfasst.

7. System nach Anspruch 6, wobei der Datenanalyseprozessor dazu ausgelegt ist, um ein Maß für die Härte aus einem Verhältnis der Verformung und Kraft als Interpretation der Widerstandsinformation abzuleiten.

8. System nach einem der vorstehenden Ansprüche, wobei der Datenanalyseprozessor dazu ausgelegt ist, um die Position von abdominalen Bereichen benachbart oder entfernt eines Fötuskörperteils basierend auf der Interpretation der Widerstandsinformationen zu bestimmen, wobei das Fötuskörperteil ein Kopf, ein Gesäß oder ein Rücken ist.

9. System nach Anspruch 8, wobei der Datenanalyseprozessor dazu ausgelegt ist, um die Fötusposition aus den bestimmten Orten zu bestimmen.

10. Verfahren zur Überwachung der Fötusposition, umfassend:
Anlegen einer Kraft- und Verformungssensoranordnung an den abdominalen Bereich einer werdenden Mutter;
Erhalten von Widerstandsinformationen einer Vielzahl von lokalen Bereichen des Abdomens als Reaktion auf einen angelegten Druck;
Interpretieren der Widerstandsinformationen in Bezug auf die Vielzahl von lokalen Bereichen; Ableiten einer Fötusposition oder -größe aus der Interpretation der Widerstandsinformation;
und
Bereitstellen einer Darstellung der Fötusposition oder der Fötusgröße.

11. Verfahren nach Anspruch 10, wobei das Erhalten von Widerstandsinformationen das Messen von Kraft und Verformung umfasst und das Verfahren das Ableiten eines Maßes für die Härte aus einem Verhältnis von Verformung und Kraft umfasst.

12. Verfahren nach Anspruch 10 oder 11, umfassend das Bestimmen der Stelle von abdominalen Bereichen benachbart oder entfernt des Fötuskörperteils basierend auf der Interpretation der Widerstandsinformationen, wobei das Fötuskörperteil eines oder mehrere von einem Kopf, einem Gesäß und einem Rücken ist und das Verfahren weiter das Bestimmen der Fötusposition von den Kopf-, Gesäß- und / oder Rückenstellen umfasst.

13. Verfahren nach einem der Ansprüche 10 bis 12, wobei die Kraft- und Verformungssensoranordnung einen Handschuh umfasst und das Verfahren einen Benutzer umfasst, der den durch den Handschuh angelegten Druck bereitstellt.

14. Verfahren nach einem der Ansprüche 10 bis 12, wobei die Kraft und Verformungssensoranordnung einen Leibgurt umfasst und das Verfahren das Bereitstellen des angelegten Drucks durch Aufblasen aufblasbarer Abschnitte des Leibgurts umfasst.

15. Verfahren nach einem der Ansprüche 10 bis 14, weiter umfassend das Ableiten einer Fötusbewegung über die Zeit und das Bereitstellen einer Darstellung der Fötusbewegung.

## Revendications

1. Système de surveillance de position fœtale comprenant :
un agencement de capteur de force et de déformation (34) pour obtenir des informations de résistance d'une pluralité de zones locales de l'abdomen en réponse à une pression appliquée, l'agencement de capteur de force et de déformation (34) comprenant un capteur de force et de déformation ;
un processeur d'analyse de données (26) pour interpréter les informations de résistance concernant la pluralité de zones locales et déduire une position fœtale ou une taille fœtale de l'interprétation ; et
une sortie (28) pour fournir des données de sortie à un dispositif de sortie afin de présenter une représentation de la position fœtale ou de la taille fœtale.

2. Système selon la revendication 1, dans lequel le processeur d'analyse de données est en outre à même de déduire le mouvement fœtal au fil du temps sur la base des positions fœtales déduites et le dispositif de sortie est en outre à même de fournir une représentation du mouvement fœtal.

3. Système selon la revendication 1 ou 2, dans lequel l'agencement de capteur de force et de déformation comprend un gant associé au capteur de force et de déformation et dans lequel le système comprend un dispositif de commande pour commander la sortie afin de guider l'utilisateur du système pour appliquer une pression à l'abdomen en utilisant le gant.

4. Système selon la revendication 1 ou 2, dans lequel l'agencement de capteur de force et de déformation comprend une sangle abdominale.

5. Système selon la revendication 4, dans lequel la sangle abdominale comprend un réseau de parties gonflables pour fournir la pression appliquée, chaque partie gonflable étant associée au capteur de force et de déformation.

6. Système selon la revendication 1, 3 ou 5, dans lequel le capteur de force et de déformation comprend un capteur de force pour mesurer la force et un capteur de déformation pour mesurer la déformation en réponse à une force appliquée.

7. Système selon la revendication 6, dans lequel le processeur d'analyse de données est à même de déduire une mesure de dureté d'un rapport de la déformation et de la force en tant qu'interprétation des informations de résistance.

8. Système selon l'une quelconque des revendications précédentes, dans lequel le processeur d'analyse de données est à même de déterminer l'emplacement de zones abdominales adjacentes ou distantes d'une partie du corps fœtal sur la base de l'interprétation des informations de résistance, dans lequel la partie du corps fœtal est une partie parmi une tête, une fesse ou un dos.

9. Système selon la revendication 8, dans lequel le processeur d'analyse de données est à même de déterminer la position fœtale à partir des emplacements déterminés.

10. Procédé de surveillance de la position fœtale, comprenant :
l'application d'un agencement de capteur de force et de déformation à la zone abdominale d'une femme enceinte ;
l'obtention d'informations de résistance d'une pluralité de zones locales de l'abdomen en réponse à une pression appliquée ;
l'interprétation des informations de résistance par rapport à la pluralité de zones locales ;
l'obtention d'une position fœtale ou d'une taille fœtale à partir de l'interprétation des informations de résistance ; et
la fourniture d'une représentation de la position fœtale ou de la taille fœtale.

11. Procédé selon la revendication 10, dans lequel l'obtention d'informations de résistance comprend la mesure de la force et de la déformation et le procédé comprend la déduction d'une mesure de dureté à partie d'un rapport de la déformation et de la force.

12. Procédé selon la revendication 10 ou 11, comprenant la détermination de l'emplacement de zones abdominales adjacentes ou écartées d'une partie du corps fœtal sur la base de l'interprétation des informations de résistance, dans lequel la partie du corps fœtal est une ou plusieurs parties parmi une tête, une fesse et un dos et le procédé comprend en outre la détermination de la position fœtale à partir des emplacements de la tête, de la fesse et/ou du dos.

13. Procédé selon l'une quelconque des revendications 10 à 12, dans lequel l'agencement de capteur de force et de déformation comprend un gant et le procédé comprend un utilisateur fournissant la pression appliquée à travers le gant.

14. Procédé selon l'une quelconque des revendications 10 à 12, dans lequel l'agencement de capteur de force et de déformation comprend une sangle abdominale et le procédé comprend la fourniture de la pression appliquée par gonflement de parties gonflables de la courroie abdominale.

15. Procédé selon l'une quelconque des revendications 10 à 14, comprenant en outre la déduction du mouvement fœtal au fil du temps et la fourniture d'une représentation du mouvement fœtal.
